# EUROPEAN PATENT SPECIFICATION

(11) **EP 4 302 755 B1**
(45) Date of publication and mention of the grant of the patent: **20.08.2025**
(21) Application number: 22183702.4
(22) Date of filing: 07.07.2022
(51) Int. Cl.: A61K 9/20, A61K 31/519, A61K 47/18

(54) **PALBOCICLIB FORMULATION CONTAINING AN AMINO ACID**
EINE AMINOSÄURE ENTHALTENDE PALBOCICLIB-FORMULIERUNG
FORMULATION DE PALBOCICLIB CONTENANT UN ACIDE AMINÉ

(43) Date of publication of application: 10.01.2024
(73) Proprietor: Lotus Pharmaceutical Co., Ltd., 11046 Taipei City (TW)
(72) Inventor: Mehta, Gaurav Bhupendrabhai, Nantou City (TW); Gattani, Yogesh Sevaramji, Nantou City (TW); Gupta, Vijender, Nantou City (TW); Chawla, Manish, Nantou City (TW)
(74) Representative: Harber IP s.r.o.

(56) References cited:
- WO-A1-2016/156070
- WO-A1-2021/220295
- WO-A1-2022/029799

## Description

### Field Of Art

The present invention relates to a pharmaceutical composition containing 6-acetyl-8-cyclopentyl-5-methyl-2-(5-piperazin-1-yl-pyridin-2-ylamino)-8H-pyrido [2,3-d pyrimidin-7-one (Palbociclib) with desirable pharmacokinetic characteristics and exhibiting favourable storage stability and dissolution properties.

### Background Art

Palbociclib is a potent and selective inhibitor of CDK4 and CDK6, having the structure:

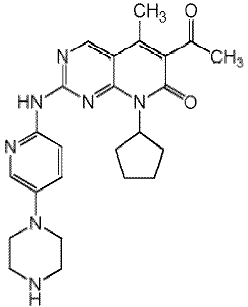

Palbociclib is a dibasic compound having two basic groups with pKa of approximately 7.3 (the secondary piperazine nitrogen) and 4.1 (the pyridine nitrogen). The solubility of palbociclib free base is pH dependent. Palbociclib is water soluble at low pH (2.1-4.5), while the solubility dramatically decreases as pH rises above 4.5. Palbociclib has poor water solubility (9 mg/mL) at pH 7.9. Concomitant administration of agents which increase gastric pH can alter the solubility and absorption of palbociclib free base formulations.

The absorption and bioavailability of a therapeutic agent can be affected by numerous factors when dosed orally, the use of certain medications, such as proton pump inhibitors (PPIs) or H2 receptor antagonists, as well as certain medical conditions. Compounds having pH-dependent solubility, particularly basic compounds, may exhibit undesirable pharmacokinetic properties, such as poor absorption and/or reduced bioavailability, which may result in significant inter-patient and intra-patient variability.

Palbociclib-free base and pharmaceutically acceptable salts thereof are disclosed in WO2003062236A. Crystalline form of Palbociclib free base having a specific surface area of ≤ 2 m²/g is described in WO2014128588A. Immediate-release capsules containing Palbociclib free base in crystalline anhydrous Form A are authorized under the brand name Ibrance^{®} (Pfizer) in EU and US. The marketed Palbociclib capsules comprise 75, 100, or 150 mg of Palbociclib in crystalline anhydrous Form A.

WO2005005426A describes an isethionate salt of Palbociclib.

Palbociclib is approved or was successfully tested for the treatment of ER+ breast cancer and HR+ breast cancer.

WO2016193860A discloses a solid dosage form comprising Palbociclib, a water-soluble acid selected from the group consisting of succinic acid, malic acid and tartaric acid, and a pharmaceutically acceptable carrier. The described solid dosage forms demonstrate excellent storage stability and provide substantially pH-independent delivery of Palbociclib with no significant food effects or adverse interactions with PPIs. WO2022029799A discloses a solid oral dosage form comprising palbociclib and at least one compound selected from the group consisting of sulphur-containing amino acid or peptide.

There still remains a need to provide improved compositions of palbociclib having improved dissolution and pharmacokinetic profiles, which also exhibit excellent storage stability.

### Detailed description of the Invention

We have surprisingly found that pharmaceutical solid compositions according to the present invention containing Palbociclib (free base and/or pharmaceutically acceptable salt) and glutamic acid or a pharmaceutically acceptable salt thereof, in combination with glucono-delta lactone, demonstrate excellent storage stability, improved dissolution profile and provide substantially pH-independent delivery of Palbociclib with no significant food effect.

The pH of the GI tract may vary based on whether a patient or subject is in a fed or fasted state. In general, the gastric residence time of a drug is longer in the presence of food than in the fasted state. If the bioavailability of a drug is significantly affected by the presence or absence of food in the GI tract, the drug is said to exhibit a "food effect". The rate of gastric emptying may also influence the concentration of drugs in solution available for absorption at different sites along the GI tract.

The present invention thus provides a pharmaceutical solid composition containing
- Palbociclib or a pharmaceutically acceptable salt thereof;
- an amino acid which is glutamic acid or a pharmaceutically acceptable salt thereof; and
- glucono-delta lactone.

Palbociclib is preferably present in the composition in the form of free base. More preferably, palbociclib is present in crystalline form. Yet more preferably, palbociclib free base crystalline form has a specific surface area (measured as BET) at least 2.3 m²/g.

Pharmaceutically acceptable salts of Palbociclib may include e. g. palbociclib isethionate salt.

The term "amino acid" refers to one of the twenty naturally occurring compounds containing amino (-NH₃⁺, or -NH₂⁺ in the case of proline) and carboxylate (-COO⁻) functional groups, attached to the same C atom (they are thus α-amino acids). Amino acids may also be used in the form of pharmaceutically acceptable salts.

Amino acids include leucine, glycine, alanine, valine, isoleucine, proline, methionine, cysteine, histidine, arginine, lysine, phenylalanine, tyrosine, tryptophan, aspartic acid, glutamic acid, serine, threonine, asparagine, glutamine, and pharmaceutically acceptable salts thereof. Preferably, the amino acid is selected from the group consisting of cysteine, glycine, proline, aspartic acid, glutamic acid, and pharmaceutically acceptable salts thereof.

Glutamic acid and salts thereof are suitable for maintaining acidic pH of the pharmaceutical composition.

| | |
|---|---|
| CAS Number: | 90-80-2 |
| IUPAC Name: | Glucono-delta-lactone |
| Molecular Formula: | |
| Structural Formula: | 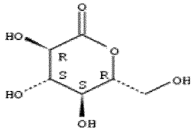 |
| Molecular Weight: | 178.14 |
| Synonyms: | EINECS Number 202-016-5 |

Glucono-delta-lactone (GDL), the inner ester of gluconic acid, is formed by the removal of water. Commercially it is produced by an anaerobic fermentation process converting a carbohydrate source into gluconic acid and glucono-delta-lactone. After fermentation, the gluconic acid is separated from GDL by crystallization. Glucono-delta-lactone (GDL) is a convenient substitute for the free acid, especially where, due to the reversibility between the acid and its lactone form, a gradual change in the pH is preferred.

In some embodiments of the invention, the solid composition contains an intragranular phase containing palbociclib or a pharmaceutically acceptable salt thereof, and an extragranular phase containing glutamic acid or pharmaceutically acceptable salt(s) thereof, and glucono-delta lactone.

In some embodiments of the invention, the solid composition contains at least two layers, the first layer containing palbociclib or a pharmaceutically acceptable salt thereof, and the second or further layer containing glutamic acid or pharmaceutically acceptable salt thereof, and glucono-delta lactone.

In some embodiments of the invention, the amount of palbociclib or a pharmaceutically acceptable salt thereof in the solid composition is within the range of 10 to 50 wt. %, preferably 17 to 23 wt. %.

In some embodiments of the invention, the amount of glutamic acid or pharmaceutically acceptable salt thereof in the solid composition is within the range of 3 to 25 wt. %, preferably 6 to 18 wt. %.

In some embodiments of the invention, the amount of glucono-delta lactone in the solid composition is within the range of 4 to 15 wt. %,-preferably 5 to 7 wt. %.

In some embodiments of the invention, the amount of Palbociclib or a pharmaceutically acceptable salt thereof in the solid composition is within the range of 10 to 50 wt. % and the amount of glutamic acid or pharmaceutically acceptable salt thereof is within the range of 3 to 25 wt. %.

In some embodiments of the invention, the amount of Palbociclib or a pharmaceutically acceptable salt thereof in the solid composition is within the range of 10 to 50 wt. %, the amount of glutamic acid or pharmaceutically acceptable salt thereof is within the range of 3 to 25 wt. % and the amount of glucono-delta lactone is within the range of 4 to 15 wt. %.

In some embodiments of the invention, the amount of Palbociclib or a pharmaceutically acceptable salt thereof in the solid composition is within the range of 17 to 23 wt. % and the amount of glutamic acid or pharmaceutically acceptable salt thereof is within the range of 6 to 18 wt. %.

In some embodiments of the invention, the amount of Palbociclib or a pharmaceutically acceptable salt thereof in the solid composition is within the range of 17 to 23 wt. %, the amount of glutamic acid or pharmaceutically acceptable salt thereof is within the range of 6 to 18 wt. % and the amount of glucono-delta lactone is within the range of 5 to 7 wt. %.

The solid composition of the invention further contains at least one pharmaceutically acceptable excipient, preferably selected from disintegrants, binders, diluents, lubricants, and glidants.

Disintegrants may be added to the pharmaceutical granulate composition to promote the breakup of the tablet or capsule into smaller fragments in an aqueous environment, thereby increasing the available surface area and promoting a more rapid release of the active pharmaceutical ingredient. Suitable examples of disintegrants to be used according to the present invention include crospovidone, sodium starch glycolate, croscarmellose sodium, and mixtures of any of the foregoing. Preferred disintegrant is sodium starch glycolate or crospovidone. Disintegrants can be added as intragranular or extragranular excipients. Disintegration occurs by the rapid uptake of water followed by rapid and enormous swelling. When added as an intragranular excipient it helps to break the granules whereas when added as an extragranular excipient it helps the tablet or capsule to disintegrate. In a preferred embodiment of the present invention, the disintegrant is added extra granularly and intragranularly.

The pharmaceutical formulation further contains a binder which is selected from the group of povidone, co-povidone, hydroxypropyl methylcellulose, hydroxy propyl cellulose, and sodium carboxyl methylcellulose.

Diluents are fillers that are used to increase the bulk volume of the pharmaceutical granulate composition. Generally, by combining a diluent with the active pharmaceutical ingredient, the final product is given adequate weight and size to assist in production and handling. Suitable examples of diluents to be used according to the present invention include starch, pregelatinized starch, microcrystalline cellulose, calcium phosphate, lactose, sorbitol, mannitol, and sucrose. According to the preferred embodiment of the invention microcrystalline cellulose and/or lactose are used as diluents.

Lubricants are preferably selected from the group containing magnesium stearate, calcium stearate, stearic acid, glyceryl behenate, hydrogenated castor oil, glycerine fumarate.

The pharmaceutical composition according to the invention is prepared by using different granulation methods, preferably selected from dry or wet granulation. Dry granulation includes dry mixing, slug-de-slug, and roller compaction processes. Wet granulation includes aqueous granulation and non-aqueous granulation. Other wet granulation technologies like Extrusion - Spherization, Spray drying, Supercritical fluid, Fluid Bed Granulation, Hotmelt Granulation, freeze granulation, Foam Granulation etc. are also suitable. Preferably, dry granulation or direct compression is used, or granulation followed by filling of capsules.

The composition form of the invention is preferably formulated as a tablet, a coated tablet or a capsule. The coated tablet may be a film-coated tablet. The tablet may be a bi-layer or a multi-layer tablet.

The solid composition of the invention is suitable for use in the treatment of cancer, more specifically in the treatment of ER+ or HR+ breast cancer.

### Brief description of Drawings

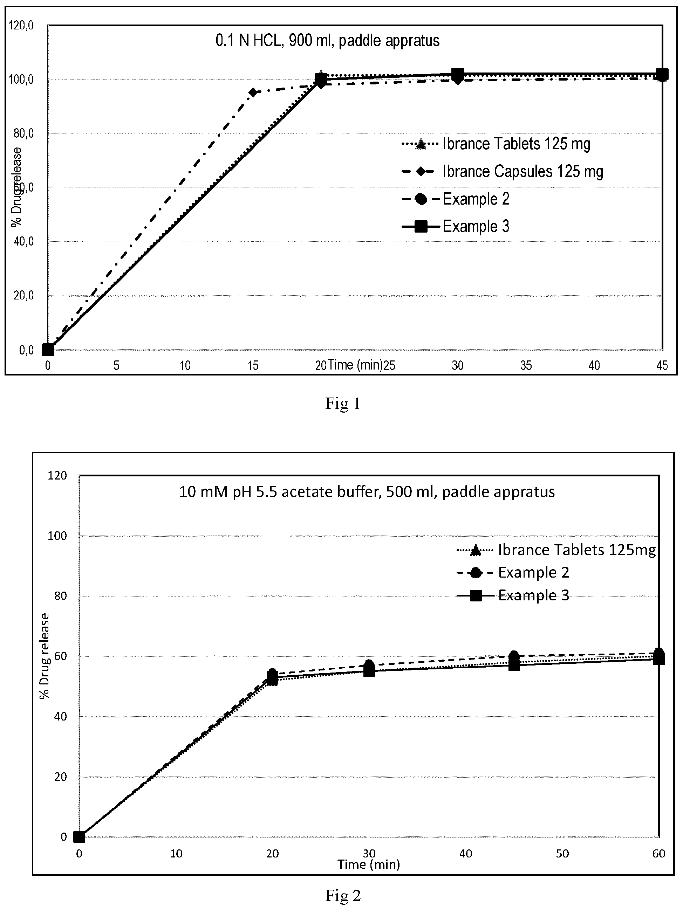

Fig 1. In vitro dissolution data comparison of the tested products from Examples 2 and 3 with the reference products Ibrance tablets at 37 °C in 0.1 N HCL in a USP 2 paddle apparatus.
Fig 2. In vitro dissolution data comparison of the tested products from Examples 2 and 3 with the reference products Ibrance tablets at 37 °C in a pH 5.5, 10 mM acetate buffer solution in a USP 2 paddle apparatus.

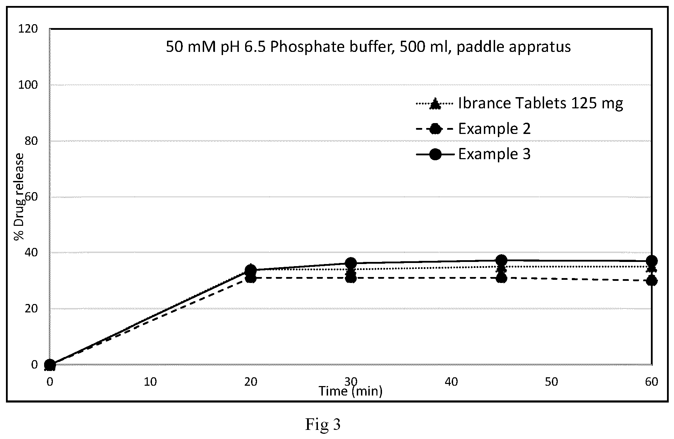

Fig 3. In vitro dissolution data comparison of the tested products from Examples 2 and 3 with the reference product Ibrance tablets at 37 °C in a pH pH 6.5, 50 mM phosphate buffer solution in a USP 2 paddle apparatus.

### Examples of carrying out the Invention

### Examples 1 - 4

Solid compositions comprising Palbociclib, one or more amino acids or salts thereof and optionally a water-soluble acid Glucono-delta-lactone (GDL) are prepared by dry granulation process.

Examples 2 and 4 do not fall into the scope of claims.

The solid composition contains Palbociclib (Free base, preferably with Specific surface area NLT 2.3 m²/g specified by BET method), Microcrystalline cellulose (trade name: MCC, FMC), Glucono-delta-lactone (GDL) (trade name: GDL, Roquette), Glutamic acid (Merck), Glutamic acid hydrochloride (Spectrum Chemicals), L-Cysteine Hydrochloride monohydrate (Spectrum), L-Cysteine Hydrochloride Anhydrous (Merck), crospovidone (trade name: VIVAPHARM ^{®} PVPP, JRS pharma), colloidal silicon dioxide (trade name: Aerosil 200 Pharma, Evonik), Magnesium stearate (From Peter Greven) according to the formulation proportions in Table 1.

The intragranular part 1-4 of the composition according to Table 1 was thoroughly mixed using a diffusion mixer, granulated by roller compaction, followed by milling through co-mill 1.2 mm and the final granules were further lubricated. The granules were mixed with the extra granular excipients according to Table 1 and compressed into tablet. The tablet was further coated using Opadry purple. The drug content is about 19 % w/w of the total weight of the coated tablet.

**Table 1**

| | **Strength: 125 mg** | **Example 1** | | **Example 2** | |
|---|---|---|---|---|---|
| **Sr No.** | **Ingredients** | **mg/Tablet** | **%w/w** | **mg/Tablet** | **%w/w** |
| **Intragranular material** | | | | | |
| **1** | Palbociclib | 125.000 | 19.23 | 125.000 | 19.23 |
| **2** | Microcrystalline cellulose | 240.000 | 36.92 | 240.000 | 36.92 |
| **3** | Colloidal silicon dioxide | 13.000 | 2.00 | 13.000 | 2.00 |
| **4** | Crosspovidone | 18.750 | 2.88 | 18.750 | 2.88 |
| **5** | Magnesium stearate | 13.000 | 2.00 | 13.000 | 2.00 |
| Weight at intragranular | | 409.750 | 63.038 | 409.750 | 63.038 |

| **Extragranular material** | | | | | |
|---|---|---|---|---|---|
| **6** | Crosspovidone | 18.750 | 2.88 | 18.750 | 2.88 |
| **7** | Glucono-delta-lactone (GDL) | 35.000 | 5.38 | -- | -- |
| **8** | Glutamic acid Hydrochloride | 30.000 | 4.62 | -- | -- |
| **9** | Cysteine Hydrochloride monohydrate | 30.000 | 4.62 | -- | -- |
| **10** | Cysteine Hydrochloride monohydrate | -- | -- | 90.000 | 13.85 |
| **11** | Glutamic acid | -- | -- | -- | -- |
| **12** | Cysteine Hydrochloride Anhydrous | -- | -- | -- | -- |
| **13** | Microcrystalline cellulose | 94.500 | 14.54 | 99.500 | 15.31 |
| **14** | Magnesium stearate | 13.000 | 2.00 | 13.000 | 2.00 |
| Total uncoated tablet weight | | 631.000 | 97.08 | 631.000 | 97.08 |
| **15** | Coating -Opadry Purple | 19.000 | 2.92 | 19.000 | 2.92 |
| Total weight of film coated tablets | | 650.000 | 100.00 | 650.000 | 100.00 |

| | **Strength: 125 mg** | **Example 3** | | **Example 4** | |
|---|---|---|---|---|---|
| **Sr No.** | **Ingredients** | **mg/Tablet** | **%w/w** | **mg/Tablet** | **%w/w** |
| **Intragranular material** | | | | | |
| **1** | Palbociclib | 125.000 | 19.23 | 125.000 | 19.23 |
| **2** | Microcrystalline cellulose | 240.000 | 36.92 | 240.000 | 36.92 |
| **3** | Colloidal silicon dioxide | 13.000 | 2.00 | 13.000 | 2.00 |
| **4** | Crosspovidone | 18.750 | 2.88 | 18.750 | 2.88 |
| **5** | Magnesium stearate | 13.000 | 2.00 | 13.000 | 2.00 |
| Weight at intragranular | | 409.750 | 63.038 | 409.750 | 63.038 |

| **Extragranular material** | | | | | |
|---|---|---|---|---|---|
| **6** | Crosspovidone | 18.750 | 2.88 | 18.750 | 2.88 |
| **7** | Glucono-delta-lactone (GDL) | 35.000 | 5.38 | 35.000 | 5.38 |
| **8** | Glutamic acid Hydrochloride | -- | -- | -- | -- |
| **9** | Cysteine Hydrochloride monohydrate | -- | -- | -- | -- |
| **10** | Cysteine Hydrochloride monohydrate | -- | -- | -- | -- |
| **11** | Glutamic acid | 100.000 | 15.38 | -- | -- |
| **12** | Cysteine Hydrochloride Anhydrous | -- | -- | 80.000 | 12.31 |
| **13** | Microcrystalline cellulose | 54.500 | 8.38 | 74.500 | 11.46 |
| **14** | Magnesium stearate | 13.000 | 2.00 | 13.000 | 2.00 |
| Total uncoated tablet weight | | 631.000 | 97.08 | 631.000 | 97.08 |
| **15** | Coating -Opadry Purple | 19.000 | 2.92 | 19.000 | 2.92 |
| Total weight of film coated tablets | | 650.000 | 100.00 | 650.000 | 100.00 |

Dissolution conditions are the following:

### Dissolution conditions in 0.1 N HCl

| **Parameters** | **Dissolution Tester Conditions** |
|---|---|
| Medium | 0.1 N HCL, 900 mL |
| Apparatus | USP II (Paddle) |
| Temperature | 37.0 ± 0.5°C |
| Sampling Time | 20/30/45 mins |
| Sampling Volume | 10 mL |

### Dissolution conditions at pH 5.5

The solid pharmaceutical compositions described in examples 2 and 3 were further evaluated for dissolution and compared with the reference product at 37°C in a pH 5.5, 10 mM acetate buffer solution in a USP 2 apparatus with paddles.

| **Parameters** | **Dissolution Tester Conditions** |
|---|---|
| Medium | 10 mM pH 5.5 acetate buffer, 500 mL |
| Apparatus | USP II (Paddle) |
| Temperature | 37.0 ± 0.5°C |
| Sampling Time | 10/15/20/30/45/60 mins |
| Sampling Volume | 10 mL |

### Dissolution conditions at pH 6.5

The solid pharmaceutical compositions described in examples 21 and 22 were further evaluated for dissolution and compared with the reference product at 37°C in a pH 6.5, 50 mM phosphate buffer solution containing 0.1 M NaCl, in a USP 2 apparatus with paddles.

| **Parameters** | **Dissolution Tester Conditions** |
|---|---|
| Medium | 50 mM pH 6.5 Phosphate buffer, 500 mL |
| Apparatus | USP II (Paddle) |
| Temperature | 37.0 ± 0.5°C |
| Sampling Time | 10/15/20/30/45/60 mins |
| Sampling Volume | 10 mL |

### Stability testing

Stability testing was performed according to the ICH Harmonised Tripartite Guideline: Stability Testing of New Drug Substances and Products Q1A(R2), Step 4 version of 6 February 2003.

| Attribute | Specifications | Results at testing Intervals Accelerated Stability condition: 40±2°C, 75±5%RH | | | |
|---|---|---|---|---|---|
| | Formulation | (Example 2) | | (Example 3) | |
| | Time (Months) | 0 | 1 | 0 | 1 |
| Assay/ Palbociclib | By HPLC method | 97.4% | 97.6% | 98.0% | 98.5% |
| Degradation product | Any unspecified impurity | ND | 0.07% | ND | ND |
| | Total impurities | ND | 0.07% | ND | ND |

## Claims

1. Solid pharmaceutical composition, containing
- palbociclib or a pharmaceutically acceptable salt thereof,
- glutamic acid or a pharmaceutically acceptable salt thereof; and
- glucono-delta lactone.

2. The composition of claim 1, wherein palbociclib is present in the composition in the form of free base.

3. The composition of claim 1, wherein palbociclib is present in the composition as a free base in crystalline form having a specific surface area (measured as BET) at least 2.3 m²/g.

4. The composition according to claim 1, wherein the glutamic acid salt is glutamic acid hydrochloride.

5. The composition according to any one of the preceding claims, wherein the amount of palbociclib or a pharmaceutically acceptable salt thereof in the solid composition is within the range of 10 to 50 wt. %, preferably 17 to 23 wt. %
and/or
wherein the amount of glutamic acid or a pharmaceutically acceptable salt thereof in the solid composition is within the range of 3 to 25 wt. %, preferably 6 to 18 wt. %
and/or
wherein the amount of glucono-delta lactone in the solid composition is within the range of 4 to 15 wt.%, preferably 5 to 7 wt. %.

6. The composition according to any one of the preceding claims, wherein the amount of palbociclib or a pharmaceutically acceptable salt thereof in the solid composition is within the range of 17 to 23 wt. % and the amount of glutamic acid or a pharmaceutically acceptable salt thereof is within the range of 6 to 18 wt.%.

7. The composition according to any one of the preceding claims, which further contains at least one pharmaceutically acceptable excipient, selected from disintegrants, binders, diluents, lubricants, and glidants.

8. The composition according to claim 7 which contains at least one disintegrant selected from sodium starch glycolate and crospovidone.

9. The solid composition according to any one of claims 1-8, which contains an intragranular phase containing palbociclib or a pharmaceutically acceptable salt thereof, and an extragranular phase containing glutamic acid or pharmaceutically acceptable salt thereof, and glucono-delta lactone.

10. The solid composition according to any one of claims 1-8, which contains at least two layers, one layer containing palbociclib or a pharmaceutically acceptable salt thereof, and a different layer containing glutamic acid or pharmaceutically acceptable salt thereof, and glucono-delta lactone.

11. The solid composition according to claim 9 or 10 which is selected from a tablet, a coated tablet and a capsule, more preferably selected from a film-coated tablet, a bi-layer tablet and a multi-layer tablet.

12. The solid composition according to any one of claims 1-11 for use in the treatment of cancer, preferably in the treatment of ER+ or HR+ breast cancer.

## Patentansprüche

1. Feste pharmazeutische Zusammensetzung, enthaltend
- Palbociclib oder ein pharmazeutisch verträgliches Salz davon,
- Glutaminsäure oder ein pharmazeutisch verträgliches Salz davon; und
- Glucono-delta-Lacton.

2. Zusammensetzung nach Anspruch 1, wobei Palbociclib in der Zusammensetzung als freie Base vorliegt.

3. Zusammensetzung nach Anspruch 1, wobei Palbociclib in der Zusammensetzung als freie Base in kristalliner Form mit einer spezifischen Oberfläche (gemessen mittels BET) von mindestens 2,3 m²/g vorliegt.

4. Zusammensetzung nach Anspruch 1, wobei das Glutaminsäuresalz Glutaminsäurehydrochlorid ist.

5. Zusammensetzung nach einem der vorhergehenden Ansprüche, wobei der Gehalt an Palbociclib oder einem pharmazeutisch verträglichen Salz davon in der festen Zusammensetzung im Bereich von 10 bis 50 Gew.-%, vorzugsweise 17 bis 23 Gew.-%, liegt,
und/oder
wobei der Gehalt an Glutaminsäure oder einem pharmazeutisch verträglichen Salz davon in der festen Zusammensetzung im Bereich von 3 bis 25 Gew.-%, vorzugsweise 6 bis 18 Gew.-%, liegt.
und/oder
wobei der Gehalt an Glucono-delta-Lacton in der festen Zusammensetzung im Bereich von 4 bis 15 Gew.-%, vorzugsweise 5 bis 7 Gew.-%, liegt.

6. Die Zusammensetzung nach einem der vorhergehenden Ansprüche, wobei der Gehalt an Palbociclib oder einem pharmazeutisch verträglichen Salz davon in der festen Zusammensetzung im Bereich von 17 bis 23 Gew.-% und der Gehalt an Glutaminsäure oder einem pharmazeutisch verträglichen Salz davon im Bereich von 6 bis 18 Gew.-% liegt.

7. Die Zusammensetzung nach einem der vorhergehenden Ansprüche, die zusätzlich mindestens einen pharmazeutisch verträglichen Hilfsstoff enthält, ausgewählt aus Sprengmitteln, Bindemitteln, Verdünnungsmitteln, Schmiermitteln und Gleitmitteln.

8. Zusammensetzung nach Anspruch 7, die mindestens ein Sprengmittel enthält, ausgewählt aus Natriumstärkeglykolat und Crospovidon.

9. Feste Zusammensetzung nach einem der Ansprüche 1-8, die eine intragranuläre Phase enthaltend Palbociclib oder ein pharmazeutisch verträgliches Salz davon und eine extragranuläre Phase enthaltend Glutaminsäure oder ein pharmazeutisch verträgliches Salz davon und Glucono-delta-Lacton enthält.

10. Feste Zusammensetzung nach einem der Ansprüche 1-8, die mindestens zwei Schichten enthält, wobei eine Schicht Palbociclib oder ein pharmazeutisch verträgliches Salz davon enthält und eine andere Schicht Glutaminsäure oder ein pharmazeutisch verträgliches Salz davon und Glucono-delta-Lacton enthält.

11. Feste Zusammensetzung nach Anspruch 9 oder 10, ausgewählt aus einer Tablette, einer überzogenen Tablette und einer Kapsel, bevorzugter ausgewählt aus einer Filmtablette, einer Zweischichttablette und einer Mehrschichttablette.

12. Die feste Zusammensetzung nach einem der Ansprüche 1-11 zur Verwendung bei der Behandlung von Krebs, vorzugsweise bei der Behandlung von ER+- oder HR+-Brustkrebs.

## Revendications

1. Composition pharmaceutique solide contenant
- du palbociclib ou un sel pharmaceutiquement acceptable de celui-ci,
- de l'acide glutamique ou un sel pharmaceutiquement acceptable de celui-ci ; et
- de la glucono-delta-lactone.

2. Composition selon la revendication 1, où le palbociclib est présent sous forme de base libre.

3. Composition selon la revendication 1, où le palbociclib est présent sous forme de base libre sous forme cristalline ayant une surface spécifique (mesurée en BET) d'au moins 2,3 m²/g.

4. Composition selon la revendication 1, où le sel d'acide glutamique est le chlorhydrate d'acide glutamique.

5. Composition selon l'une quelconque des revendications précédentes, où la quantité de palbociclib ou d'un sel pharmaceutiquement acceptable de celui-ci dans la composition solide est de 10 à 50 % en poids, de préférence de 17 à 23 % en poids,
et/ou
la quantité d'acide glutamique ou d'un sel pharmaceutiquement acceptable de celui-ci dans la composition solide est de 3 à 25 % en poids, de préférence de 6 à 18 % en poids,
et/ou
la quantité de glucono-delta-lactone dans la composition solide est de 4 à 15 % en poids, de préférence de 5 à 7 % en poids.

6. Composition selon l'une quelconque des revendications précédentes, où la quantité de palbociclib ou d'un sel pharmaceutiquement acceptable de celui-ci dans la composition solide est de 17 à 23 % en poids et la quantité d'acide glutamique ou d'un sel pharmaceutiquement acceptable de celui-ci est de 6 à 18 % en poids.

7. Composition selon l'une quelconque des revendications précédentes, contenant en outre au moins un excipient pharmaceutiquement acceptable, choisi parmi les agents désintégrants, les liants, les diluants, les lubrifiants et les agents glissants.

8. Composition selon la revendication 7, contenant au moins un désintégrant choisi parmi le glycolate d'amidon sodique et la crospovidone.

9. Composition solide selon l'une quelconque des revendications 1 à 8, contenant une phase intragranulaire contenant du palbociclib ou un sel pharmaceutiquement acceptable de celui-ci, et une phase extragranulaire contenant de l'acide glutamique ou un sel pharmaceutiquement acceptable de celui-ci, et de la glucono-delta-lactone.

10. Composition solide selon l'une quelconque des revendications 1 à 8, contenant au moins deux couches: une couche contenant du palbociclib ou un sel pharmaceutiquement acceptable de celui-ci, et une autre couche contenant de l'acide glutamique ou un sel pharmaceutiquement acceptable de celui-ci, et de la glucono-delta-lactone.

11. Composition solide selon la revendication 9 ou 10, choisie parmi un comprimé, un comprimé enrobé et une capsule, de préférence choisie parmi un comprimé pelliculé, un comprimé bicouche et un comprimé multicouche.

12. Composition solide selon l'une quelconque des revendications 1 à 11 pour utilisation dans le traitement du cancer, de préférence dans le traitement du cancer du sein ER+ ou HR+.
